(19) Europäisches Patentamt
European Patent Office
Office européen des brevets

(11) EP 0 655 234 B1

(12) **FASCICULE DE BREVET EUROPEEN**

(45) Date de publication et mention de la délivrance du brevet: **28.10.1998 Bulletin 1998/44**

(51) Int. Cl.[6]: **A61K 7/032**, A61K 7/48, A61K 7/00

(21) Numéro de dépôt: **94402689.7**

(22) Date de dépôt: **24.11.1994**

(54) **Composition cosmétique pour le maquillage sous forme d'un mascara contenant au moins une cire et un pseudo-latex**

Schminkzusammensetzung in Maskaraform enthaltend mindestens ein Wachs und ein Pseudo-latex

Make-up composition in mascara form containing at least a wax and a pseudo-latex

(84) Etats contractants désignés:
**AT BE CH DE ES FR GB IT LI NL SE**

(30) Priorité: **24.11.1993 FR 9314051**

(43) Date de publication de la demande:
**31.05.1995 Bulletin 1995/22**

(73) Titulaire: **L'OREAL, S.A.**
**75008 Paris (FR)**

(72) Inventeurs:
- **Piot, Bertrand**
**F-92250 La Garenne-Colombes (FR)**
- **Debert, Danièle**
**F-91600 Savigny Sur Orge (FR)**
- **Patraud, Jeanne**
**F-75013 Paris (FR)**

(74) Mandataire:
**Stalla-Bourdillon, Bernard**
**Cabinet Nony**
**29, rue Cambacérès**
**75008 Paris (FR)**

(56) Documents cités:

| | |
|---|---|
| EP-A- 0 477 053 | EP-A- 0 530 084 |
| EP-A- 0 557 196 | EP-A- 0 573 229 |
| WO-A-91/12793 | FR-A- 2 528 699 |

EP 0 655 234 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Printed by Xerox (UK) Business Services
2.16.3/3.4

## Description

La présente invention a pour objet une composition cosmétique pour le maquillage notamment des cils, dite composition de mascara, contenant l'association d'au moins une cire et d'un pseudo-latex.

Il est d'usage courant de réaliser des compositions de mascara contenant au moins une cire, toutefois, celle-ci n'est jamais utilisée seule, car le maquillage avec de telles compositions s'avère très médiocre conduisant à la formation sur les cils d'un film non homogène ce qui se traduit par la formation de pellicules craquantes, immédiatement après le séchage.

Afin d'y remédier, il a été proposé dans les brevets français FR 83.09997 (2.528.699) et FR 84.17661 (2.573.305), l'utilisation conjointe d'au moins une cire et d'un polymère filmogène présent en solution dans la phase aqueuse.

Il a également été proposé dans la demande de brevet PCT WO/92/21316, des compositions de traitement des cheveux et des cils contenant l'association d'une silicone, d'un latex et d'un agent de mise en suspension du latex et de la silicone et/ou d'un épaississant.

Il a par ailleurs été proposé des compositions de mascara ne contenant pas de cire. Ainsi, il a été décrit dans la demande de brevet japonais KOKAI 57-62216, une composition de mascara aqueuse contenant comme agent filmogène un latex de synthèse.

Si ces compositions ont permis une certaine amélioration de la qualité des produits de maquillage, on a maintenant constaté de façon surprenante et inattendue qu'en utilisant l'association d'au moins une cire et d'un pseudo-latex particulier constitué de particules d'un polymère à fonctions acides carboxyliques partiellement neutralisées, on obtenait des compositions de mascara présentant d'excellentes qualités cosmétiques. En effet, après application, elles augmentent significativement l'allongement et le recourbement des cils, et sont en outre éliminables à l'eau.

On rappelle que par l'expression "pseudo-latex", on désigne une suspension constituée de particules généralement sphériques d'un polymère, celles-ci étant obtenues par dispersion du polymère dans une phase aqueuse appropriée.

L'expression "pseudo-latex" ne doit pas être confondue avec l'expression "latex" ou "latex synthétique" qui est également une suspension constituée de particules d'un polymère qui sont obtenues directement par polymérisation d'un ou plusieurs monomères dans une phase aqueuse appropriée.

Plus précisément, la présente invention a pour objet une composition de mascara contenant en mélange,

a) un pseudo-latex constitué de particules d'un polymère filmogène à fonctions acides carboxyliques ayant un diamètre moyen compris entre 10 et 300 nm, ledit polymère étant choisi parmi :

(i) les copolymères acétate de vinyle/acide crotonique polyoxyéthylénés,
(ii) les copolymères acétate de vinyle/acide crotonique,
(iii) les terpolymères acétate de vinyle/acide crotonique/néodécanoate de vinyle,
(iv) les copolymères N-ocrylacrylamide/méthacrylate de méthyle/méthacrylate d'hydroxypropyle/acide acrylique/méthacrylate de tert-butylaminoéthyle,
(v) les copolymères alternés méthylvinyléther/anhydride maléique monoestérifiés par le butanol,
(vi) les terpolymères acide acrylique/acrylate d'éthyle/N-tert-butylacrylamide, et
(vii) les polymères répondant à la formule générale suivante :

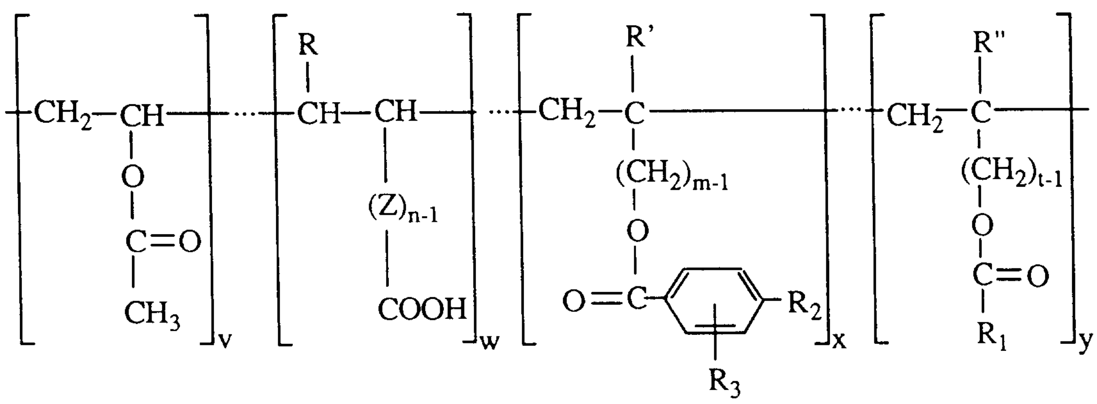

(I)

dans laquelle :

R, R' et R", identiques ou différents, représentent un atome d'hydrogène ou un radical méthyle,
m, n et t sont 1 ou 2,
$R_1$ représente un radical alkyle, linéaire ou ramifié, saturé ou insaturé ayant de 2 à 21 atomes de carbone,
$R_2$ représente un atome d'hydrogène, un radical méthyle, éthyle, tert-butyle, éthoxy, butoxy ou dodécyloxy,
$R_3$ représente un atome d'hydrogène, un radical alkyle ayant de 1 à 4 atomes de carbone ou un radical alkoxy ayant de 1 à 4 atomes de carbone,
Z représente un radical divalent pris dans le groupe constitué par :
$-CH_2-$, $CH_2-O-CH_2-$ et $-CH_2-O-(CH_2)_2-$,
v représente de 10 à 91 % et de préférence de 36 à 84% en poids,
w représente de 3 à 20 % et de préférence de 6 à 12 % en poids,
x représente de 4 à 60 % et de préférence de 6 à 40 % en poids, et
y représente de 0 à 40 % et de préférence de 4 à 30 % en poids,
v + w + x + y étant égal à 100%,

les fonctions acides carboxyliques dudit polymère étant neutralisées à un taux de neutralisation compris entre 10 et 80 % à l'aide d'un agent monobasique non volatil, ledit agent étant utilisé seul, et

ledit pseudo-latex étant présent en une proportion comprise entre 0,8 et 20 % et de préférence entre 1 et 10 % en poids de matière sèche par rapport au poids total de la composition, et

b) au moins une cire ayant un point de fusion compris entre 60°C et 110°C, et de préférence entre 65°C et 100°C, en une proportion comprise entre 2 et 40 % en poids par rapport au poids total de la composition.

De préférence, le rapport en poids entre le pseudo-latex neutralisé exprimé en poids de matière sèche et la cire est compris entre 0,025 et 2, et plus particulièrement entre 0,05 et 1.

Parmi les copolymères acétate de vinyle/acide crotonique polyoxyéthylénés, on peut notamment citer l'"Aristoflex A" d'indice d'acide 56 de la Société Hoechst.

Comme copolymères acétate de vinyle/acide crotonique, on peut citer le "Luviset CA66" d'indice d'acide 65 (acétate de vinyle/acide crotonique 90/10) de la Société BASF.

Parmi les terpolymères acétate de vinyle/acide crotonique/néodécanoate de vinyle, on peut citer la "Résine 28-29-30" d'indice d'acide 65 de la Société National Starch.

Comme copolymères N-octylacrylamide/méthacrylate de méthyle/méthacrylate d'hydroxypropyle/acide acrylique/méthacrylate de tert-butylaminoéthyle, on peut citer l'"Amphomer LV 71" d'indice d'acide 137 de la Société National Starch.

Parmi les copolymères alternés méthylvinyléther/anhydride maléique monoestérifiés par le butanol, on peut mentionner le "Gantrez ES425" d'indice d'acide 260 (méthylvinyléther/ anhydride maléique 50/50) de la Société GAF.

Comme terpolymères acide acrylique/acrylate d'éthyle/N-tert-butylacrylamide, on peut citer en particulier l'"Ultrahold 8" d'indice d'acide 62 de la Société BASF.

Comme copolymères de formule (I), on peut citer ceux décrits dans le brevet français n° 78.30596 (2.439.798) et en particulier les copolymères suivants :

- acétate de vinyle/ acide crotonique/ tert-butyl-4-benzoate de vinyle (65/10/25),
- acétate de vinyle/ acide crotonique/ tert-butyl-4-benzoate de vinyle/ néodécanoate de vinyle (57/10/25/8),
- acétate de vinyle/ acide crotonique/ tert-butyl-4-benzoate de vinyle/ néodécanoate de vinyle (70/10/10/10),
- acétate de vinyle/ acide crotonique/ benzoate de vinyle/ néodécanoate de vinyle (70/10/10/10),
- acétate de vinyle/ acide crotonique/ tert-butyl-4-benzoate de vinyle/ stéarate d'allyle (70/10/10/10).

Les polymères filmogènes à fonctions acides carboxyliques tels que définis ci-dessus sont des polymères synthétiques, insolubles dans l'eau, ayant de préférence un poids moléculaire moyen compris entre 5 000 et 700 000 mesuré par exemple en chromatographie d'exclusion stérique.

Le pseudo-latex des compositions cosmétiques selon l'invention est obtenu selon les méthodes connues de préparation des pseudo-latex sous réserve toutefois de certaines particularités qui seront mentionnées ci-après.

Le procédé général de préparation des pseudo-latex consiste à dissoudre un polymère insoluble dans l'eau dans un solvant organique, soluble ou partiellement soluble dans l'eau, à disperser sous agitation la solution ainsi obtenue dans de l'eau et à procéder ensuite à l'élimination du solvant organique par évaporation sous vide ce qui conduit à une suspension constituée de particules du polymère dont la taille est généralement inférieure au μm.

Selon ce procédé général, l'emploi d'un tensio-actif, d'un mélange de tensio-actifs ou d'un polymère colloïde protecteur ou encore d'un mélange tensio-actif/polymère colloïde protecteur est indispensable, en vue d'obtenir une bonne stabilisation des particules.

Les polymères filmogènes à fonctions acides carboxyliques tels que définis ci-dessus ne peuvent être utilisés tels

quels dans la préparation des pseudo-latex mais doivent être neutralisés à un taux de neutralisation inférieur à 100 % en vue d'éviter leur totale solubilisation dans l'eau.

Par une neutralisation partielle des polymères, on a constaté qu'il était possible d'obtenir des pseudo-latex particulièrement stables en l'absence de stabilisant hydrophile ou de tensio-actif ou encore de colloïde protecteur.

Le taux de neutralisation des polymères filmogènes à fonctions acides carboxyliques doit donc être parfaitement déterminé de telle sorte qu'ils restent insolubles dans l'eau tout en étant solubles dans le solvant organique.

Il va de soi que le taux limite supérieur de neutralisation qu'il conviendra de ne pas excéder pour que le polymère reste insoluble dans l'eau sera fonction de la nature de chaque polymère filmogène à fonctions acides carboxyliques. De façon générale, ce taux de neutralisation est généralement compris entre 30 et 80 %, et de préférence entre 40 et 70 % si le polymère a moins de 2 meq/g de fonctions acides carboxyliques et entre 10 et 50 %, de préférence entre 10 et 40 %, si le polymère a plus de 2 meq/g de fonctions acides carboxyliques.

Selon l'invention la neutralisation des fonctions acides carboxyliques est réalisée à l'aide d'un agent mono-basique non volatil choisi par exemple parmi une base minérale telle que la soude ou la potasse ou parmi un aminoalcool pris dans le groupe constitué par l'amino-2 méthyl-2 propanol-1 (AMP), la triéthanolamine, la triisopropanolamine (TPA), la monoéthanolamine, la diéthanolamine, la tri [(hydroxy-2) propyl-1] amine, l'amino-2 méthyl-2 propanediol-1,3 (AMPD) et l'amino-2 hydroxyméthyl-2 propanediol-1,3.

Dans la préparation du pseudo-latex, utilisé dans les compositions selon l'invention, la neutralisation des fonctions acides carboxyliques du polymère filmogène est réalisée *in situ* dans la solution du polymère dans le solvant organique par addition de la quantité déterminée du composé mono-basique non volatil. Le solvant organique uàlisé doit être un solvant volatil ou un mélange de tels solvants présentant un point d'ébullition inférieur à celui de l'eau et être miscible ou partiellement miscible à l'eau.

Le solvant organique tel que défini ci-dessus est de préférence choisi parmi l'acétone, la méthyléthylcétone, le tétrahydrofuranne, l'acétate de méthyle, l'acétate d'éthyle, l'isopropanol et l'éthanol.

Après l'obtention de la solution du polymère partiellement neutralisé dans le solvant organique, on procède alors à la préparation d'une émulsion en versant sous agitation, à la solution organique obtenue, une quantité appropriée d'eau contenant éventuellement un agent anti-mousse dont le rôle sera de faciliter l'évaporation ultérieure de la phase organique.

Selon une variante du procédé tel que défini ci-dessus la neutralisation des fonctions acides carboxyliques du polymère en solution dans le solvant organique peut être réalisée lors de la formation de l'émulsion en versant une solution aqueuse contenant la quantité requise du composé mono-basique non volatil.

Lors de la formation de l'émulsion l'agitation est de préférence réalisée à l'aide d'un disperseur cisaillant du type Moritz ou Ultra-Turrax ou Raineri équipé de pales défloculantes.

L'émulsion ainsi obtenue est particulièrement stable sans qu'il soit nécessaire d'employer un agent tensio-actif dans la mesure où les groupes carboxylates du polymère se placent à l'interface avec l'eau et protègent les gouttelertes de la coalescence par répulsion électrostatique.

Après formation de l'émulsion à une température comprise entre la température ambiante et 70°C environ, on procède alors à l'évaporation sous pression réduite du solvant organique jusqu'à son élimination totale, l'évaporation étant de préférence réalisée sous léger chauffage.

On obtient ainsi un pseudo-latex, c'est-à-dire une dispersion aqueuse de particules du polymère filmogène, qui est exempt de tout tensio-actif ou autre stabilisant hydrophile tout en restant très stable.

La concentration en poids du polymère filmogène sous forme de particules dans le pseudo-latex ainsi obtenu est généralement comprise entre 5 et 50 % et de préférence entre 10 et 25 % par rapport au poids total du pseudo-latex.

La taille moyenne des particules est comprise entre 10 et 300 nm mais est de préférence inférieure à 250 nm.

La polydispersité en taille des particules est relativement faible selon ce procédé de préparation du pseudo-latex, celle-ci mesurée en diffusion quasi-élastique de lumière est généralement comprise entre 0,1 et 0,40 et de préférence inférieure à 0,35.

On peut introduire dans les pseudo-latex utilisés dans les compositions selon l'invention, en vue d'améliorer leur propriétés cosmétiques et mécaniques, un agent plastifiant en une proportion comprise entre 5 et 40 % et de préférence entre 10 et 30 % en poids par rapport au poids du polymère filmogène, ledit agent se distribuant selon son coefficient de partage entre les particules et la phase aqueuse du pseudo-latex.

L'agent plastifiant qui peut être du type hydrophile ou hydrophobe, est de préférence introduit en mélange dans le solvant organique lors de la préparation du pseudo-latex, et notamment lorsqu'il est du type hydrophobe.

Lorsque l'agent plastifiant est du type hydrophile, il peut être introduit après formation du pseudo-latex dans la phase aqueuse.

Parmi les agents plastifiants pouvant être utilisés dans les compositions selon l'invention, on peut citer :

- les "Carbitols" de la Société Union Carbide à savoir le "Carbitol" ou diéthylène glycol éthyléther, le "méthyl Carbitol" ou diéthylène glycol méthyléther, le "butyl Carbitol" ou diéthylène glycol butyléther ou encore l'"hexyl Carbitol" ou

diéthylène glycol hexyléther,

- les "Cellosolves" de la Société Union Carbide à savoir le "Cellosolve" ou éthylène glycol éthyléther, le "butyl Cellosolve" ou éthylène glycol butyléther, l'"hexyl Cellosolve" ou éthylène glycol hexyléther,
- les dérivés de propylène glycol et en particulier le propylène glycol phényléther, le propylène glycol diacétate, le dipropylène glycol butyléther, le tripropylène glycol butyléther, ainsi que les "Dowanols" de la Société Dow Chemical à savoir le "Dowanol PM" ou propylène glycol méthyléther, le "Dowanol DPM" ou dipropylène glycol métliyléther et le "Dowanol TPM" ou tripropylène glycol méthyléther.

On peut encore citer :

- le diéthylène glycol méthyléther ou "Dowanol DM" de la Société Dow Chemical,
- l'huile de ricin oxyéthylénée à 40 moles d'oxyde d'éthylène telle que celle vendue par la Société Rhône Poulenc sous la dénomination de "Mulgofen EL-719",
- l'alcool benzylique,
- le citrate de triéthyle vendu par la Société Pfizer sous la dénomination de "Citroflex-2",
- le 1,3-butylène glycol,
- les phtalates et adipates de diéthyle, de dibutyle et de diisopropyle,
- les tartrates de diéthyle et de dibutyle,
- les phosphates de diéthyle, de dibutyle et de diéthyl-2 hexyle, et
- les esters de glycérol tels que le diacétate de glycérol (diacétine) et le triacétate de glycérol (triacétine).

On utilise de préférence un agent plastifiant choisi dans le groupe constitué par le dipropylène glycol méthyléther, le tripropylène glycol méthyléther, l'adipate de diéthyle et l'adipate de diisopropyle.

Les cires utilisées dans les compositions de mascara selon l'invention sont choisies parmi les cires, solides et rigides, d'origine animale, végétale, minérale ou de synthèse et leurs mélanges.

La dureté de ces cires, mesurée par la méthode de pénétration d'une aiguille est généralement comprise entre 3 et 40.

Cette méthode décrite dans les normes NFT 004 et ASTM D5, normes respectivement française et américaine, consiste à mesurer, à une température de 25°C, la profondeur de pénétration, exprimée en dixième de millimètre, d'une aiguille normalisée (pesant 2,5 g et placée dans un porte-aiguille pesant 47,5 g, soit au total 50 g) placée sur la cire pendant 5 secondes.

Parmi les cires animales, on peut citer la cire d'abeilles, la cire de lanoline, et les cires d'insectes de Chine.

Parmi les cires végétales, on peut notamment citer la cire de riz, la cire de Carnauba, la cire de Candelilla, la cire d'Ouricurry, la cire de fibres de liège, la cire de canne à sucre, la cire du Japon et la cire de Sumac.

Parmi les cires minérales, on peut notamment citer la cire de Montan, les cires microcristallines, les paraffines et l'ozokérite.

Parmi les cires de synthèse, on peut notamment citer les cires de polyéthylène, les cires obtenues par la synthèse de Fisher et Tropsch et les copolymères cireux ainsi que leurs esters.

On peut également utiliser dans les compositions selon l'invention, les cires obtenues par hydrogénation catalytique d'huiles animales ou végétales ayant des chaînes grasses, linéaires, ou ramifiées, en $C_8$-$C_{32}$.

Parmi celles-ci, on peut notamment citer l'huile de jojoba hydrogénée, l'huile de tournesol hydrogénée, l'huile de ricin hydrogénée, l'huile de coprah hydrogénée et l'huile de lanoline hydrogénée.

Les compositions de mascara selon l'invention peuvent en outre contenir des pigments.

Ces pigments peuvent être organiques ou minéraux ou peuvent être également des pigments nacrés. De tels pigments sont bien connus et sont en particulier décrits dans le brevet FR 83.09997 (2.528.699).

La proportion en pigments dans les compositions de mascara selon l'invention est généralement comprise entre 3 et 25 % en poids par rapport au poids total de la composition suivant la coloration et l'intensité de coloration recherchées.

Les compositions de mascara selon l'invention peuvent se présenter sous différentes formes. Elles peuvent en particulier se présenter sous forme d'émulsions huile-dans-eau ou eau-dans-huile ou sous forme de dispersions.

Selon une forme de réalisation préférée des compositions de mascara selon l'invention, celles-ci se présentent sous forme d'émulsions contenant au moins un agent tensio-actif anionique ou non ionique en une proportion comprise entre 2 et 30 % en poids par rapport au poids total de la composition.

Parmi les agents tensio-actifs anioniques pouvant être utilisés seuls ou en mélange, on peut citer notamment les sels alcalins, les sels d'ammonium, les sels d'amine ou les sels d'amino-alcool des composés suivants :

- les alcoylsulfates, alcoyléther sulfates, alcoylamide sulfates, éther sulfates, alcoylarylpolyéther sulfates, monoglycéride sulfates,

- les alcoylsulfonates, alcoylamide sulfonates, alcoylarylsulfonates, $\alpha$-oléfines sulfonates, paraffines sulfonates,
- les alcoylsulfosuccinates, alcoyléther sulfosuccinates, les alcoylamide sulfosuccinates,
- les alcoylsulfosuccinamates,
- les alcoylsulfoacétates, alcoylpolyglycérol carboxylates,
- les alcoylphosphates, alcoyléther phosphates,
- les N-acylsarcosinates, N-acylpolypeptidates, les acyliséthionates, et N-acyltaurates.

On entend par les termes alcoyle et acyle utilisés ci-dessus une chaîne ayant généralement de 12 à 18 atomes de carbone.

On peut citer également comme agents tensio-actifs anioniques utilisables dans les compositions selon l'invention des sels d'acides gras, tels que ceux de l'acide oléïque, ricinoléique, palmitique, stérique, les acides d'huile de coprah ou d'huile de coprah hydrogénée, et en particulier les sels d'amines tels que les stéarates d'amines.

On peut encore citer comme agents tensio-actifs anioniques les acyl lactylates dont le radical acyle comprend de 8 à 20 atomes de carbone, et les acides carboxyliques d'éthers polyglycoliques répondant à la formule :

$$R_4\text{-}(OCH_2\text{-}CH_2)_n\text{-}OCH_2\text{-}COOH$$

dans laquelle :

$R_4$ représente un radical alkyle linéaire ayant de 12 à 18 atomes de carbone et n est un nombre entier compris entre 5 et 15, et les sels desdits acides. On utilise de préférence comme agent tensio-actif anionique des stéarates d'amines.

Parmi les agents tensio-actifs non ioniques pouvant être utilisés seuls ou en mélange dans les compositions de mascara selon l'invention, on peut citer notamment les alcools, les alcoylphénols et les acides gras polyéthoxylés, polypropoxylés ou polyglycérolés à chaîne grasse ayant de 8 à 18 atomes de carbone.

On peut également citer des copolymères d'oxydes d'éthylène et de propylène, des condensats d'oxydes d'éthylène et de propylène sur des alcools gras, des amides gras polyéthoxylés, des amines grasses polyéthoxylées, des éthanolamides, des esters d'acides gras de glycol, des esters d'acides gras du sorbitan oxyéthylénés ou non, des esters d'acides gras du saccharose, des esters d'acides gras des polyéthylèneglycols, des triesters phosphoriques et des esters d'acides gras de dérivés du glucose.

On peut également citer les produits de condensation d'un monoalcool, d'un alpha-diol, d'un alcoylphénol, d'un amide ou d'un diglycolamide avec le glycidol ou un précurseur du glycidol tel que décrit dans le brevet français FR 71.17206(2.091.516), de formule :

$$R_5\text{-}CHOH\text{-}CH_2\text{-}O\text{-}(CH_2\text{-}CHOH\text{-}CH_2\text{-}O)_p\text{-}H$$

dans laquelle :

$R_5$ représente un radical aliphatique, cycloaliphatique, ou arylaliphatique ayant de préférence entre 7 et 21 atomes de carbone, les chaînes aliphatiques pouvant comporter des groupements éther, thioéther ou hydroxyméthylène, et p est un nombre entier compris entre 1 et 10.

On peut en outre citer les composés décrits dans le brevet FR 1.477.048 de formule :

$$R_6O\text{-}[C_2H_3O\text{-}(CH_2OH)]_q\text{-}H$$

dans laquelle :

$R_6$ représente un radical alcoyle, alcényle ou alcoylaryle, et q est une valeur statistique comprise entre 1 et 10.

On peut encore citer les composés décrits dans le brevet français FR 76.31975 (2.328.763) de formule :

$$R_7CONH\text{-}CH_2\text{-}CH_2O\text{-}CH_2\text{-}CH_2O\text{-}(CH_2\text{-}CHOH\text{-}CH_2\text{-}O)_r\text{-}H$$

dans laquelle :

$R_7$ représente un radical aliphatique linéaire ou ramifié, saturé ou insaturé, pouvant comporter éventuellement un

ou plusieurs groupement(s) hydroxyle(s), ayant entre 8 et 30 atomes de carbone, d'origine naturelle ou synthétique, et r est un nombre entier ou décimal compris entre 1 et 5 et désigne le degré de condensation moyen.

On utilise de préférence comme agent tensio-actif non ionique un mélange d'huile(s) et/ou d'alcool gras ou bien d'alcools polyéthoxylés ou polyglycérolés tels que les alcools stéarylique ou cétylstéarylique polyéthoxylés.

Les compositions de mascara selon l'invention peuvent comprendre en outre au moins un additif conventionnel choisi parmi un adoucissant, un conservateur, un agent séquestrant, un parfum, un agent épaississant, une huile, une silicone, un agent de cohésion, un agent alcalinisant ou acidifiant, un polymère hydrosoluble et une charge.

Les agents épaississants utilisables dans les compositions de mascara selon l'invention peuvent être d'origine naturelle ou synthétique.

Parmi les agents épaississants d'origine naturelle, on peut citer notamment les gommes de diverses sortes telles que les gommes arabique, de guar ou de caroube.

Parmi les épaississants d'origine synthétique, on peut citer notamment les dérivés cellulosiques comme l'hydroxyéthylcellulose et la carboxyméthylcellulose, les dérivés de l'amidon, les dérivés d'éthers de cellulose possédant des groupes ammonium quaternaires, les polysaccharides cationiques, les sels de polymères acryliques ou méthacryliques, les polyènes et les polysiloxanes.

On peut également obtenir un épaississement des compositions de mascara selon l'invention par adjonction d'un mélange de polyéthylène glycol et de stéarate et/ou de distéarate de polyéthylène glycol ou d'un mélange d'esters phosphoriques et d'amides gras.

Parmi les polymères hydrosolubles pouvant être utilisés dans les compositions de mascara selon l'invention, on peut citer notamment les dérivés de protéines d'origine animale ou végétale et plus particulièrement les dérivés de kératine tels que les hydrolysats de kératine et les kératines sulfoniques, la polyvinylpyrrolidone, les copolymères vinyliques tels que le copolymère de l'éther méthylvinylique et de l'anhydride maléïque ou le copolymère de l'acétate de vinyle et de l'acide crotonique, les glycoaminoglycanes, l'acide hyaluronique et ses dérivés et l'acide désoxyribonucléique et ses sels.

Parmi les charges pouvant être utilisées dans les compositions de mascara selon l'invention on peut citer notamment celles décrites dans la demande de brevet FR 91.10791 (2.680.681).

On va maintenant donner à titre d'illustration plusieurs exemples de préparation des pseudo-latex ainsi que des exemples de mascara selon l'invention.

## EXEMPLES DE PREPARATION DE PSEUDO-LATEX

**EXEMPLE I** : *Préparation du pseudo-latex du copolymère acétate de vinylelacide crotonique/tert-butyl-4-benzoate de vinyle (65/10/25) neutralisé à 50 % d'après son indice d'acide*

La préparation de ce copolymère est décrite à l'exemple 19 du brevet français n° 78.30596 (2.439.798) et se présente sous forme de billes ayant un diamètre de 0,5 à 1 mm.

40 g du copolymère défini ci-dessus (indice d'acide : 65) sont additionnés petit à petit sous agitation à une solution homogène de 110 g d'acétone, 2,07 g d'amino-2 méthyl-2 propanol-1 (quantité correspondante à 50 % de neutralisation d'après l'indice d'acide) et 8 g de monométhyléther de tripropylène glycol.

Après agitation à température ambiante pendant 30 minutes, la dissolution du polymère est totale.

A la phase organique ainsi obtenue, on ajoute en 5 minutes environ sous agitation à l'aide d'un disperseur cisaillant du type Ultra-Turrax à 2000 tr/min, une phase aqueuse pour réaliser l'émulsion, celle-ci étant constituée de 109,54 g d'eau permutée et 0,456 g d'un agent anti-mousse siliconé "Burst RSD 10".

Après la fin de l'addition de la phase aqueuse, à température ambiante, on poursuit l'agitation pendant 10 à 15 min, ce qui permet de conduire à l'obtention d'une émulsion translucide et stable.

On procède alors à la concentration à l'aide d'un évaporateur rotatif sous vide partiel à une température inférieure à 45°C. Après élimination totale de l'acétone, on obtient une dispersion stable laiteuse et peu visqueuse.

La taille des particules a été mesurée en diffusion quasi-élastique de lumière au Coulter modèle M4 et a donné les résultats suivants :

Taille moyenne des particules : 67 nm
Facteur de polydispersité : 0,22.

**EXEMPLE II** : *Préparation du pseudo-latex du copolymère N-octylacrylamidel méthacrylate de méthylelméthacrylate d'hydroxypropylelacide acryliquelméthacrylate de tert- butylaminoéthyle ("Amphomer LV 71" de la Société National Starch) neutralisé à 30 % d'après son indice d'acide*

Selon un mode opératoire analogue à celui décrit précédemment, on prépare à température ambiante, un pseudolatex à partir de 150 g du copolymère "Amphomer LV 71" additionnés dans une solution homogène de 1063 g de tétrahydrofuranne, 9,80 g d'amino-2 méthyl-2-propanol-1 (quantité correspondante à 30 % de neutralisation d'après l'indice d'acide du copolymère "Amphomer LV 71") et 30 g de monométhyléther de tripropylène glycol, et d'une phase aqueuse constituée de 812 g d'eau permutée et 1,71 g d'un agent anti-mousse siliconé "Burst RSD 10".

On obtient ainsi un pseudo-latex dont la concentration en polymère est de 15 % en poids par rapport au poids total de la composition.

La taille des particules a été mesurée en diffusion quasi-élastique de lumière au Coulter modèle M4 et a donné les résultats suivants :

Taille moyenne des particules : 299 nm.
Facteur de polydispersité : 0,33.

**EXEMPLE III** : *Préparation du pseudo-latex du copolymère acétate de vinylelacide crotonique 90/10 ("Luviset CA 66" de la Société B.A.S.F.) neutralisé à 50 % d'après son indice d'acide*

60 g du copolymère "Luviset CA 66" sous forme de poudre (indice d'acide : 65) sont additionnés petit à petit sous agitation à une solution homogène de 22,5 g de méthyléthylcétone, 3,5 g d'amino-2 méthyl-2 propanol-1 (AMP) (quantité correspondante à 50 % de neutralisation d'après l'indice d'acide du copolymère "Luviset CA 66") et 12 g de monométhyléther de tripropylène glycol.

Après poursuite de l'agitation pendant 30 minutes, la dissolution du copolymère est totale.

A la phase organique ainsi obtenue, on ajoute sous agitation à l'aide d'un disperseur Moritz à 2500 tr/min une phase aqueuse, pour réaliser l'émulsion, celle-ci étant constituée par 325 g d'eau permutée et 0,69 g d'un agent anti-mousse siliconé "Burst RSD10".

L'addition de la phase aqueuse est réalisée en environ 15 minutes puis on maintient l'agitation à 3000 tr/min pendant 15 minutes. On obtient ainsi une émulsion d'aspect laiteux et moyennement visqueuse.

On procède alors à l'évaporation de tout le solvant organique à l'aide d'un évaporateur rotatif sous vide partiel et chauffage à environ 40-45°C. L'évaporation est poursuivie jusqu'à élimination totale de la méthyléthylcétone en respectant l'azéotrope avec l'eau.

La quantité d'eau éliminée par formation de l'azéotrope est ensuite rajoutée à la dispersion pour obtenir un pseudolatex ayant une concentration en polymère filmogène de 15 %. Le pseudo-latex obtenu est stable, d'un aspect opaque légèrement bleuté et moyennement visqueux.

La taille des particules a été mesurée en diffusion quasi-élastique de lumière au Coulter modèle M4 et a donné les résultats suivants :

Taille moyenne des particules : 247 nm
Facteur de polydispersité : 0,47.

**EXEMPLES DE MASCARA**

**EXEMPLE 1** : *Mascara crème*

| PARTIE A | |
|---|---|
| - Stéarate de triéthanolamine | 12,0 g |
| - Cire d'abeilles | 6,0 g |
| - Cire de Carnauba | 1,0 g |
| - Paraffine | 3,5 g |

| PARTIE B | |
|---|---|
| - Oxydes de fer noir | 6,0 g |

| PARTIE C | |
|---|---|
| - Hydroxyéthylcellulose commercialisée sous la dénomination de "Cellosize QP" par la Société Amerchol | 1,0 g |
| - Gomme arabique | 2,0 g |
| - Hydrolysat de kératine | 1,8 g |

| PARTIE D | |
|---|---|
| - Pseudo-latex de l'exemple I | 5,0 g |
| - Conservateurs qs | |
| - Eau qsp | 100 g |

Ce mascara est obtenu en portant les ingrédients de la partie A à 85°C, à laquelle on ajoute la partie B et l'on agite à l'aide d'une turbine.

On fait ensuite bouillir l'eau de la préparation, ajoute les conservateurs, puis à 85°C, les ingrédients de la partie C.

On ajoute alors la phase aqueuse obtenue (85°C) à la partie A (80°C) sous agitation à l'aide d'une turbine (émulsification à 30°C) puis on ajoute enfin le pseudo-latex de la partie D et agite à l'aide d'une pale.

**EXEMPLE 2** : *Mascara*

On prépare, selon le même mode opératoire qu'à l'exemple 1, un mascara ayant la composition suivante :

| PARTIE A | |
|---|---|
| - Stéarate de triéthanolamine | 8,0 g |
| - Stéarate de glycéryle commercialisé sous la dénomination de "Geleol" par la Société Gattefosse | 3,0 g |
| - Cire d'abeilles | 8,0 g |
| - Cire de Carnauba | 2,0 g |
| - Paraffine | 5,0 g |

| PARTIE B | |
|---|---|
| - Oxydes de fer noir | 5,0 g |

| PARTIE C | |
|---|---|
| - Hydroxyéthylcellulose commercialisée sous la dénomination de "Cellosize QP" par la Société Amerchol | 1,2 g |
| - Gomme arabique | 2,0 g |

| PARTIE D | |
|---|---|
| - Pseudo-latex de l'exemple III | 7,0 g |
| - Conservateurs qs | |
| - Eau qsp | 100 g |

**EXEMPLE 3** : *Mascara*

On prépare, selon le même mode opératoire qu'à l'exemple 1, un mascara ayant la composition suivante :

| PARTIE A | |
|---|---|
| - Stéarate de triéthanolamine | 12,0 g |
| - Cire d'abeilles | 5,0 g |
| - Cire de Carnauba | 1,5 g |
| - Paraffine | 4,0 g |

| PARTIE B | |
|---|---|
| - Noir de carbone | 3,0 g |

| PARTIE C | |
|---|---|
| - Hydroxyéthylcellulose commercialisée sous la dénomination de "Cellosize QP" par la Société Amerchol | 0,8 g |
| - Gomme arabique | 2,0 g |
| - Hydrolysat de kératine commercialisé sous la dénomination de "Kerasol" par la Société Croda | 2,0 g |

| PARTIE D | |
|---|---|
| - Pseudo-latex de l'exemple II | 13,3 g |
| - Conservateurs qs | |
| - Eau qsp | 100 g |

On a comparé l'effet cosmétique de ce mascara obtenu en remplaçant dans la même composition, le pseudo-latex de l'exemple II par la même quantité d'une solution à 15 % du polymère filmogène "Amphomer LV 71" neutralisé à 100 % d'après son indice d'acide par l'amino-2 méthyl-2 propanol-1. Lorsqu'on utilise le mascara selon l'invention, les cils après maquillage sont nettement plus longs et présentent une courbure plus prononcée que les cils traités à l'aide du mascara de comparaison.

## Revendications

**1.** Composition de mascara caractérisée par le fait qu'elle contient en mélange :

a) un pseudo-latex constitué de particules d'un polymère filmogène à fonctions acides carboxyliques ayant un diamètre moyen compris entre 10 et 300 nm, ledit polymère étant choisi parmi :

(i) les copolymères acétate de vinyle/acide crotonique polyoxyéthylénés,
(ii) les copolymères acétate de vinyle/acide crotonique,
(iii) les terpolymères acétate de vinyle/acide crotonique/néodécanoate de vinyle,
(iv) les copolymères N-octylacrylamide/méthacrylate de méthyle/méthacrylate d'hydroxypropyle/acide acrylique/méthacrylate de tert-butylaminoéthyle,
(v) les copolymères alternés méthylvinyléther/anhydride maléique monoestérifiés par le butanol,
(vi) les terpolymères acide acrylique/acrylate d'éthyle/N-tert-butylacrylamide, et
(vii) les polymères répondant à la formule générale suivante :

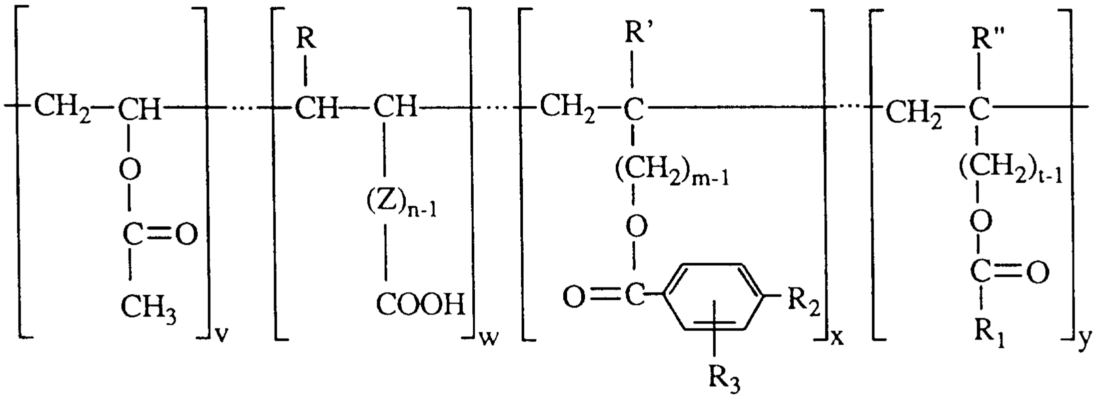

(I)

dans laquelle :

R, R' et R", identiques ou différents, représentent un atome d'hydrogène ou un radical méthyle,
m, n et t sont 1 ou 2,
$R_1$ représente un radical alkyle, linéaire ou ramifié, saturé ou insaturé ayant de 2 à 21 atomes de carbone,
$R_2$ représente un atome d'hydrogène, un radical méthyle, éthyle, tert-butyle, éthoxy, butoxy ou dodécyloxy,
$R_3$ représente un atome d'hydrogène, un radical alkyle de 1 à 4 atomes de carbone ou un radical alkoxy de 1 à 4 atomes de carbone,
Z représente un radical divalent pris dans le groupe constitué par :
$-CH_2-$, $CH_2-O-CH_2-$ et $CH_2-O-(CH_2)_2-$,
v représente de 10 à 91% et de préférence de 36 à 84% en poids,
w représente de 3 à 20 % et de préférence de 6 à 12 % en poids,
x représente de 4 à 60 % et de préférence de 6 à 40% en poids, et
y représente de 0 à 40 % et de préférence de 4 à 30 % en poids,
v + w + x + y étant égal à 100%,

les fonctions acides carboxyliques dudit polymère étant neutratisées à un taux de neutralisation compris entre 10 et 80 % à l'aide d'un agent monobasique non volatil, ledit agent étant utilisé seul, et

ledit pseudo-latex étant présent en une proportion comprise entre 0,8 et 20 % et de préférence entre 1 et 10 % en poids de matière sèche par rapport au poids total de la composition, et

b) au moins une cire ayant un point de fusion compris entre 60°C et 110°C, et de préférence entre 65°C et 100°C, en une proportion comprise entre 2 et 40 % en poids par rapport au poids total de la composition.

**2.** Composition de mascara selon la revendication 1 caractérisée par le fait que le rapport en poids entre le pseudolatex neutralisé exprimé en poids de matière sèche et la cire est compris entre 0,025 et 2.

**3.** Composition de mascara selon l'une quelconque des revendications précédentes, caractérisée par le fait que le polymère filmogène à fonctions acides carboxyliques a un poids moléculaire moyen compris entre 5 000 et 700.000.

**4.** Composition de mascara selon l'une quelconque des revendications précédentes caractérisée par le fait que le polymère filmogène à fonctions acides carboxyliques est neutralisé à l'aide d'un agent mono-basique non volatil choisi parmi : la soude, la potasse, l'amino-2 méthyl-2 propanol-1, la triéthanolamine, la triisopropanolamine, la monoéthanolamine, la diéthanolamine, la tri[(hydroxy-2) propyl-1] amine, l'amino-2 méthyl-2 propanediol-1,3 et l'amino-2 hydroxyméthyl-2 propanediol-1,3.

**5.** Composition de mascara selon l'une quelconque des revendications précédentes, caractérisée par le fait que si ledit polymère filmogène a moins de 2meq/g de fonctions acides carboxyliques le taux de neutralisation est compris entre 30 et 80 % et de préférence entre 40 et 70 %.

6. Composition de mascara selon l'une quelconque des revendications 1 à 4, caractérisée par le fait que si ledit polymère filmogène a plus de 2meq/g de fonctions acides carboxyliques le taux de neutralisation est compris entre 10 et 50 % et de préférence entre 10 et 40 %.

7. Composition de mascara selon l'une quelconque des revendications précédentes caractérisée par le fait que le pseudo-latex contient un agent plastifiant en une proportion comprise entre 5 et 40 % en poids par rapport au poids du polymère filmogène, ledit agent plastifiant étant distribué selon son coefficient de partage entre les particules et la phase aqueuse du pseudo-latex.

8. Composition de mascara selon l'une quelconque des revendications précédentes caractérisée par le fait que ladite cire est choisie dans le groupe constitué par la cire d'abeilles, la cire de lanoline, la cire d'insectes de Chine, la cire de riz, la cire de Carnauba, la cire de Candelilla, la cire d'Ouricurry, la cire de fibres de liège, la cire de canne à sucre, la cire du Japon, la cire de Sumac, la cire de Montan, les cires microcristallines, les paraffines, l'ozokérite, les cires de polyéthylène et les huiles hydrogénées.

9. Composition de mascara selon l'une quelconque des revendications précédentes caractérisée par le fait qu'elle contient en outre des pigments en une proportion comprise entre 3 et 25 % en poids par rapport au poids total de la composition.

10. Composition de mascara selon l'une quelconque des revendications précédentes caractérisé par le fait qu'elle se présente sous forme d'une émulsion huile-dans-eau ou eau-dans-huile ou d'une dispersion.

11. Composition de mascara selon la revendication 10, caractérisée par le fait qu'elle se présente sous forme d'une émulsion contenant au moins un agent tensio-actif anionique ou non-ionique en une proportion comprise entre 2 et 30 % en poids par rapport au poids total de la composition.

12. Composition de mascara selon l'une quelconque des revendications précédentes caractérisée par le fait qu'elle contient en outre au moins un additif conventionnel choisi parmi un adoucissant, un conservateur, un agent séquestrant, un parfum, un agent épaississant, une huile, une silicone, un agent de cohésion, un agent alcalinisant ou acidifiant, un polymère hydrosoluble et une charge.

13. Utilisation d'une composition de mascara selon l'une quelconque des revendications précédentes pour le maquillage des cils en vue de les allonger et/ou les recourber.

14. Procédé de maquillage des cils caractérisé par le fait qu'il consiste à appliquer sur les cils une composition de mascara selon l'une quelconque des revendications 1 à 12.

## Claims

1. Mascara composition, characterized in that it contains, as a mixture:

   a) a pseudolatex consisting of particles of a film-forming polymer containing carboxylic acid functional groups having a mean diameter of between 10 and 300 nm, the said polymer being chosen from:

      (i) vinyl acetate/polyoxyethylenated crotonic acid copolymers,
      (ii) vinyl acetate/crotonic acid copolymers,
      (iii) vinyl acetate/crotonic acid/vinyl neodecanoate terpolymers,
      (iv) N-octylacrylamide/methyl methacrylate/hydroxypropyl methacrylate/acrylic acid/tert-butylaminoethyl methacrylate copolymers,
      (v) methyl vinyl ether/maleic anhydride monoesterified with butanol alternating copolymers,
      (vi) acrylic acid/ethyl acrylate/N-tert-butylacrylamide terpolymers, and
      (vii) polymers corresponding to the following general formula:

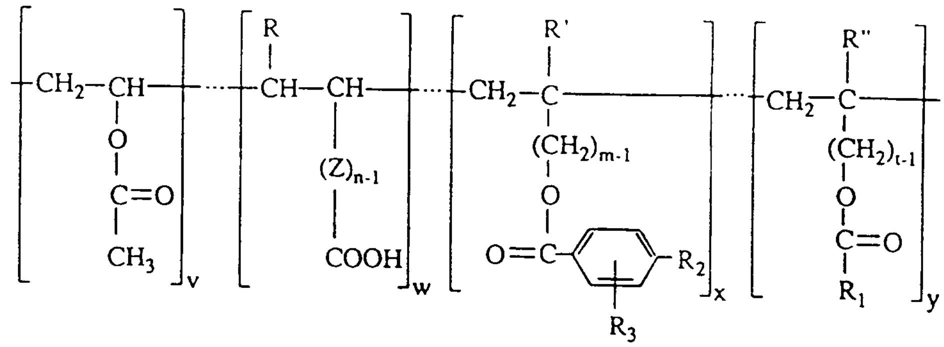

(I)

in which:

R, R' and R", which are identical or different, represent a hydrogen atom or a methyl radical,
m, n and t are 1 or 2,
$R_1$ represents a saturated or unsaturated, linear or branched, alkyl radical having from 2 to 21 carbon atoms,
$R_2$ represents a hydrogen atom or a methyl, ethyl, tert-butyl, ethoxy, butoxy or dodecyloxy radical,
$R_3$ represents a hydrogen atom, an alkyl radical containing 1 to 4 carbon atoms or an alkoxy radical having from 1 to 4 carbon atoms,
Z represents a divalent radical taken from the group consisting of:
$-CH_2-$, $-CH_2-O-CH_2-$ and $-CH_2-O-(CH_2)_2-$,
v represents from 10 to 91% and preferably from 36 to 84% by weight,
w represents from 3 to 20% and preferably from 6 to 12% by weight,
x represents from 4 to 60% and preferably from 6 to 40% by weight, and
y represents from 0 to 40% and preferably from 4 to 30% by weight,
$v + w + x + y$ being equal to 100%,

the carboxylic acid functional groups of the said polymer being neutralized to a degree of neutralization of between 10 and 80% using a non-volatile monobasic agent, the said agent being used alone, and
the said pseudolatex being present in a proportion of between 0.8 and 20% and preferably between 1 and 10% by weight of solids with respect to the total weight of the composition, and
b) at least one wax having a melting point of between 60°C and 110°C, and preferably between 65°C and 100°C, in a proportion of between 2 and 40% by weight with respect to the total weight of the composition.

2. Mascara composition according to Claim 1, characterized in that the ratio by weight between the neutralized pseudolatex, expressed by weight of solids, and the wax is between 0.025 and 2.

3. Mascara composition according to either of the preceding claims, characterized in that the film-forming polymer containing carboxylic acid functional groups has a mean molecular weight of between 5,000 and 700,000.

4. Mascara composition according to any one of the preceding claims, characterized in that the film-forming polymer containing carboxylic acid functional groups is neutralized using a non-volatile monobasic agent chosen from: sodium hydroxide, potassium hydroxide, 2-amino-2-methyl-1-propanol, triethanolamine, triisopropanolamine, monoethanolamine, diethanolamine, tri(2-hydroxy-1-propyl)amine, 2-amino-2-methyl-1,3-propanediol and 2-amino-2-hydroxymethyl-1,3-propanediol.

5. Mascara composition according to any one of the preceding claims, characterized in that, if the said film-forming polymer has less than 2 meq/g of carboxylic acid functional groups, the degree of neutralization is between 30 and 80% and preferably between 40 and 70%.

6. Mascara composition according to any one of Claims 1 to 4, characterized in that, if the said film-forming polymer has more than 2 meq/g of carboxylic acid functional groups, the degree of neutralization is between 10 and 50% and preferably between 10 and 40%.

**7.** Mascara composition according to any one of the preceding claims, characterized in that the pseudolatex contains a plasticizing agent in a proportion of between 5 and 40% by weight with respect to the weight of the film-forming polymer, the said plasticizing agent being distributed according to its partition coefficient between the particles and the aqueous phase of the pseudolatex.

**8.** Mascara composition according to any one of the preceding claims, characterized in that the said wax is chosen from the group consisting of beeswax, lanolin wax, Chinese insect wax, rice wax, carnauba wax, candelilla wax, ouricury wax, cork fibre wax, sugar cane wax, Japan wax, sumach wax, montan wax, microcrystalline waxes, paraffins, ozocerite, polyethylene waxes and hydrogenated oils.

**9.** Mascara composition according to any one of the preceding claims, characterized in that it additionally contains pigments in a proportion of between 3 and 25% by weight with respect to the total weight of the composition.

**10.** Mascara composition according to any one of the preceding claims, characterized in that it is provided in the form of an oil-in-water or water-in-oil emulsion or of a dispersion.

**11.** Mascara composition according to Claim 10, characterized in that it is provided in the form of an emulsion containing at least one anionic or non-ionic surface-active agent in a proportion of between 2 and 30% by weight with respect to the total weight of the composition.

**12.** Mascara composition according to any one of the preceding claims, characterized in that it additionally contains at least one conventional additive chosen from an emollient, a preserving agent, a sequestering agent, a fragrance, a thickening agent, an oil, a silicone, a cohesion agent, a basifying or acidifying agent, a water-soluble polymer and a filler.

**13.** Use of a mascara composition according to any one of the preceding claims for making up the eyelashes with a view to lengthening them and/or to bending them.

**14.** Method for making up the eyelashes, characterized in that it consists in applying to the eyelashes a mascara composition according to any one of Claims 1 to 12.

**Patentansprüche**

**1.** Mascara-Zubereitung, dadurch gekennzeichnet, daß sie als Gemisch enthält:

a) einen Pseudolatex, bestehend aus Teilchen eines filmbildenden Polymeren mit Carbonsäurefunktionen mit einem mittleren Durchmesser zwischen 10 und 300 nm, wobei das Polymer ausgewählt ist unter:

i) polyoxyethylenierten Copolymeren von Vinylacetat und Crotonsäure,
ii) Vinylacetat / Crotonsäure-Copolymeren,
iii) Terpolymeren von Vinylacetat / Crotonsäure / Vinylneodecanoat,
iv) Copolymeren von N-Octylacrylamid / Methylmethacrylat / Hydroxypropylmethacrylat / Acrylsäure / tert.-Butylaminoethylmethacrylat,
v) alternierenden Copolymeren von Methylvinylether / Maleinsäureanhydrid, einfach verestert mit Butanol,
vi) Terpolymeren von Acrylsäure / Ethylacrylat / N-tert.-butylacrylamid, und
vii) Polymeren entsprechend der allgemeinen Formel:

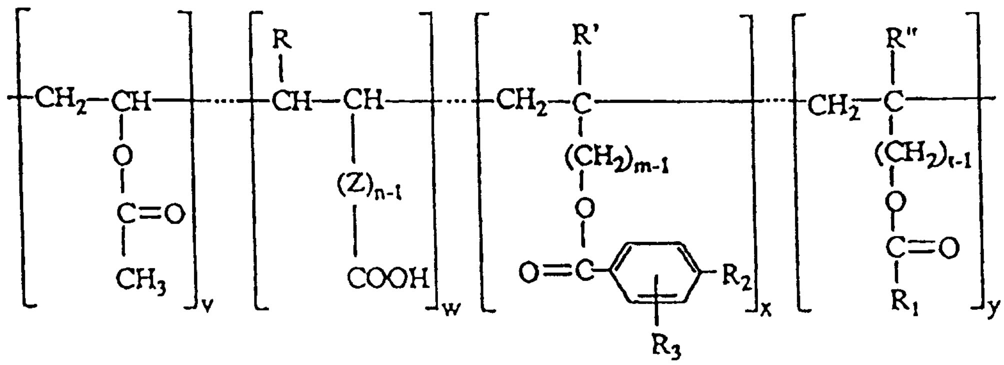
(I)

in der:

R, R' und R", die gleich oder verschieden sein können, ein Wasserstoffatom oder ein Methylrest darstellen,
m, n und t 1 oder 2 bedeuten,
$R_1$ einen linearen oder verzweigten, gesättigten oder ungesättigten Alkylrest mit 2 bis 21 Kohlenstoffatomen darstellt,
$R_2$ ein Wasserstoffatom, einen Methyl-, Ethyl-, tert.-Butyl-, Ethoxy, Butoxy- oder Dodecyloxyrest darstellt,
$R_3$ ein Wasserstoffatom, einen Alkylrest mit 1 bis 4 Kohlenstoffatomen oder einen Alkoxyrest mit 1 bis 4 Kohlenstoffatomen darstellt,
Z einen zweiwertigen Rest aus der Gruppe: $-CH_2-$, $-CH_2-O-CH_2-$und $-CH_2-O-(CH_2)_2-$ bedeutet,
v für 10 bis 91 %, vorzugsweise 36 bis 84 Gew.-%,
w für 3 bis 20 %, vorzugsweise 6 bis 12 Gew.-%,
x für 4 bis 60 %, vorzugsweise 6 bis 40 Gew.-%, und
y für 0 bis 40 %, vorzugsweise 4 bis 30 Gew.-% steht,

wobei v + w + x + y gleich 100 % ist,
wobei die Carbonsäurefunktionen des Polymeren mit Hilfe eines monobasischen, nichtflüchtigen Mittels auf einen Neutralisationsgrad zwischen 10 und 80 % neutralisiert sind, wobei dieses Mittel als einziges verwendet wird, und
der Pseudolatex in einer Menge zwischen 0,8 bis 20 %, vorzugsweise zwischen 1 und 10 Gew.- % der Trockenbestandteile, bezogen auf das Gesamtgewicht der Zubereitung, vorhanden ist, und
b) mindestens ein Wachs mit einem Schmelzpunkt zwischen 60°C und 110 °C, vorzugsweise zwischen 65 °C und 100 °C, in einer Menge zwischen 2 und 40 Gew.-%, bezogen auf das Gesamtgewicht der Zubereitung.

2. Mascara-Zubereitung gemäß Anspruch 1, dadurch gekennzeichnet, daß das Gewichtsverhältnis zwischen dem neutralisierten Pseudolatex, ausgedrückt als Gewicht der Trockenbestandteile, und dem Wachs zwischen 0,025 und 2 liegt.

3. Mascara-Zubereitung gemäß einem der vorstehenden Ansprüche, dadurch gekennzeichnet, daß das filmbildende Polymer mit Carbonsäurefunktionen ein mittleres Molekulargewicht zwichen 5.000 und 700.000 aufweist.

4. Mascara-Zubereitung gemäß einem der vorstehenden Ansprüche, dadurch gekennzeichnet, daß das filmbildende Polymer mit Carbonsäurefunktionen mit Hilfe eines monobasischen, nichtflüchtigen Mittels neutralisiert ist, das ausgewählt ist unter: Soda, Kali, 2-Amino-2-methyl-1-propanol, Triethanolamin, Triisopropanolamin, Monoethanolamin, Diethanolamin, Tri-[(2-hydroxy)-1-propyl]-amin, 2-Amino-2-methyl-1,3-propandiol und 2-Amino-2-hydroxymethyl-1,3-propandiol.

5. Mascara-Zubereitung gemäß einem der vorstehenden Ansprüche, dadurch gekennzeichnet, daß das filmbildende Polymer mindestens 2 mÄq/g Carbonsäurefunktionen hat, wobei der Neutralisationsgrad zwischen 30 und 80 % und vorzugsweise zwischen 40 und 70 % liegt.

6. Maskara-Zubereitung gemäß einem der Ansprüche 1 bis 4, dadurch gekennzeichnet, daß das filmbildende Polymer über 2 mÄq/g Carbonsäurefunktionen aufweist, wobei der Neutralisationsgrad zwischen 10 und 50 % und vorzugsweise zwischen 10 und 40 % liegt.

7. Mascara-Zubereitung gemäß einem der vorstehenden Ansprüche, dadurch gekennzeichnet, daß der Pseudolatex einen Weichmacher in einer Menge zwischen 5 und 40 Gew.- %, bezogen auf das Gewicht des filmbildenden Polymeren, enthält, wobei der Weichmacher gemäß seines Verteilungskoeffizienten zwischen den Teilchen und der wäßrigen Phase des Pseudolatex verteilt ist.

8. Mascara-Zubereitung gemäß einem der vorstehenden Ansprüche, dadurch gekennzeichnet, daß das Wachs aus der Gruppe gewählt ist, die aus Bienenwachs, Lanolinwachs, Chinawachs (chinesisches Insektenwachs), Reiswachs, Carnaubawachs, Candelillawachs, Ouricurrywachs, Korkfaserwachs, Zuckerrohrwachs, Japanwachs, Sumacwachs, Montanwachs, mikrokristallinen Wachsen, Paraffinen, Ozokerit, Polyethylenwachsen und hydrierten Ölen besteht.

9. Mascara-Zubereitung gemäß einem der vorstehenden Ansprüche, dadurch gekennzeichnet, daß sie zusätzlich Pigmente in einer Menge zwischen 3 und 25 Gew.-%, bezogen auf das Gesamtgewicht der Zubereitung, enthält.

10. Mascara-Zubereitung gemäß einem der vorstehenden Ansprüche, dadurch gekennzeichnet, daß sie in Form einer Öl-in-Wasser- oder Wasser-in-Öl-Emulsion oder einer Dispersion vorliegt.

11. Mascara-Zubereitung gemäß Anspruch 10, dadurch gekennzeichnet, daß sie in Form einer Emulsion vorliegt, die mindestens ein anionisches oder nichtionisches oberflächenaktives Mittel in einer Menge zwischen 2 und 30 Gew.-% enthält, bezogen auf das Gesamtgewicht der Zubereitung.

12. Mascara-Zubereitung gemäß einem der vorstehenden Ansprüche, dadurch gekennzeichnet, daß sie zusätzlich mindestens ein herkömmliches Additiv enthält, das ausgewählt ist unter einem Weichmacher, einem Konservierungsmittel, einem Sequestrationsmittel, einem Parfüm, einem Verdickungsmittel, einem Öl, einem Silikon, einem Kohäsionsmittel, einem Mittel zum Alkalisieren oder Ansäuern, einem wasserlöslichen Polymeren sowie einem Zuschlag.

13. Verwendung einer Mascara-Zubereitung gemàß einem der vorstehenden Ansprüche zum Schminken der Wimpern zum Verlängern und/oder Biegen.

14. Verfahren zum Schminken der Wimpern, dadurch gekennzeichnet, daß es darin besteht, auf die Wimpern eine Mascara-Zubereitung gemäß einem der Ansprüche 1 bis 12 aufzubringen.